# FASCICULE DE BREVET EUROPEEN

(11) **EP 0 904 039 B1**
(45) Date de publication et mention de la délivrance du brevet: **31.01.2001**
(21) Numéro de dépôt: 97926050.2
(22) Date de dépôt: 29.05.1997
(51) Int. Cl.: A61F 13/08

(54) **ORTHESE COMPRESSIVE DU TYPE COLLANT DE CONTENTION, NOTAMMENT DESTINEE A LA PERIODE DU POST-PARTUM**
KOMPRESSIONSORTHESE IN FORM EINER STÜTZSTRUMPFHOSE, INSBESONDERE FÜR DIE ZEIT NACH DER GEBURT
COMPRESSIVE SUPPORT ORTHOSIS TIGHTS, PARTICULARLY FOR THE POST-PARTUM PERIOD

(30) Priorité: 30.05.1996 FR 9606672
(43) Date de publication de la demande: 31.03.1999
(73) Titulaire: Innothera Topic International, 94110 Arcueil (FR)
(72) Inventeur: GARDON-MOLLARD, Christian, F-63400 Chamalières (FR)
(74) Mandataire: Dupuis-Latour, Dominique
(86) Numéro de dépôt international: FR9700933
(87) Numéro de publication internationale: WO9745080

(56) Documents cités:
- DE-U- 7 515 833
- US-A- 4 106 514

## Description

L'invention a trait au domaine des orthèses compressives des membres inférieurs, généralement connues sous la dénomination "collants de contention".

Ces collants de contention permettent de réaliser une compression forte des membres inférieurs avec une pression dégressive depuis la cheville, et sont indiqués dans les diverses symptomatologies d'insuffisance veineuse. Ils sont réalisés en un matériau élastique, typiquement une maille tricotée de texture très serrée, avec un dimensionnement très précis du collant pour réaliser la pression dégressive recherchée.

Dans la mesure où l'on cherche à réaliser une compression des membres inférieurs, allant en diminuant au fur et à mesure que l'on se rapproche du haut de la jambe, la partie abdominale est une partie à élasticité relativement faible, ne réalisant pas de compression significative (contention) du ventre.

On cherche souvent, en particulier pour les collants de grossesse (comme dans le DE-U-7515833), à réduire l'élasticité de la zone ventrale pour donner à cette partie du collant une plus grande déformabilité et permettre ainsi au collant de s'adapter convenablement aux modifications de volume de l'abdomen pendant la grossesse.

Au contraire, l'invention a pour objet un collant de contention qui réalise une compression significative du ventre (donc peu déformable dans cette région), combinée à la compression des membres inférieurs, utile notamment pendant la rééducation abdominale après l'accouchement (post-partum).

Plus précisément, l'orthèse compressive de l'invention présente les caractéristiques énoncées par la revendication 1. Les revendications 2 et 3 visent deux mises en oeuvre possibles.

Une telle structure permet d'exercer sur le ventre une pression constante et d'apporter à la patiente une aide et un confort permanent durant la période de la rééducation qui suit l'accouchement.

On va maintenant décrire un exemple de réalisation de l'invention, en référence aux dessins annexés.

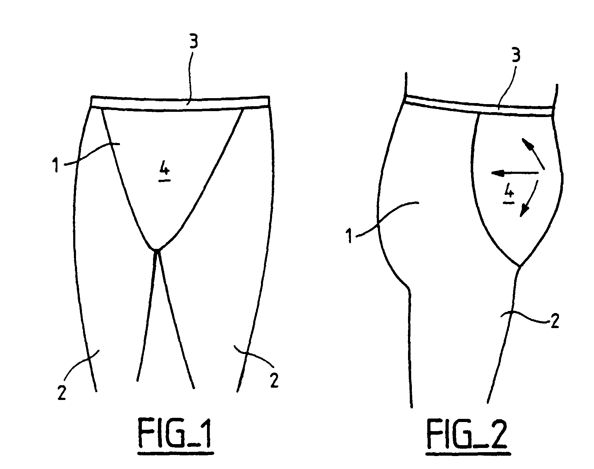

La figure 1 est une vue de face du collant de contention selon l'invention.

La figure 2 est une vue de côté de ce même collant.

Sur les figures, on a schématisé une orthèse compressive en forme de collant de contention comprenant une partie ventrale 1 en maille compressive et deux parties de jambe 2 attenantes, également en maille compressive.

La maille est une maille classique, l'invention étant applicable à toutes les structures de mailles connues en elles-mêmes du spécialiste des techniques de tricotage (tramée, jersey, côtes, micromesh pincée ou flottée, etc.).

Un tel type de maille est par exemple utilisé pour les bas et collants de contention *Varisma* (marque déposée) d'Innothéra Topic.

Les matériaux utilisés peuvent être un élasthanne guipé coton et polyamide, un élasthanne guipé polyamide sans coton, ou encore un mélange d'élasthanne et d'élasto-diène (latex de caoutchouc).

Pour permettre l'application d'une pression significative au niveau du ventre, on prévoit, dans une région sensiblement triangulaire délimitée par la ceinture 3 et les aines, une pièce rapportée 4 à forte élasticité, par exemple un tissu du type tissu élastique compressif référence n° 13638-6 de la société Elastic (Catteins, Autriche).

Le tissu rapporté peut être un tricot, une structure chaîne et trame, armure tissée, etc.

En variante, on peut réaliser en maille (tricotage d'un renfort élastique) cette partie à forte élasticité 4.

La caractéristique principale de la partie 4, qu'elle soit assemblée ou tricotée, est une élasticité à forte pression dans les deux sens, de manière que cette pièce se comporte à la manière d'un plastron.

Le tricotage de la partie 4 peut être réalisé suivant tous les types de maillage connus, avec ou sans fil vanisé (c'est-à-dire avec inclusion d'un fil supplémentaire pendant la réalisation de la maille).

L'article ainsi réalisé procure une compression combinée du ventre et des membres inférieurs, compression combinée qui est particulièrement indiquée dans le cas d'une symptomatologie d'insuffisance veineuse induite par une grossesse, ou concomitante à la période précédant et suivant l'accouchement.

Pendant la période de rééducation abdominale après l'accouchement, la compression du ventre aura pour effet de renforcer la ceinture abdominale distendue par la grossesse, en venant s'opposer à la déformation naturelle de l'abdomen.

## Revendications

1. Une orthèse compressive du type collant de contention, comprenant une partie ventrale (1) en maille compressive et deux parties de jambe (2) attenantes, également en maille compressive, la partie ventrale comportant à l'avant une région (4) de forme sensiblement triangulaire s'étendant entre la ceinture et les aines,
caractérisée en ce que ladite région (4) s'étendant entre la ceinture et les aines est une région peu déformable formant plastron, présentant une élasticité à forte pression dans les deux sens,
de sorte que cette région exerce sur l'abdomen pendant la période du post-partum une pression sensiblement constante venant s'opposer à la déformation de l'abdomen, en combinaison avec une compression vasculaire des membres inférieurs par les parties de jambe.

2. L'orthèse compressive de la revendication 1, dans laquelle la région à élasticité accrue est formée par ajout d'une pièce de tissu à élasticité bidimensionnelle renforcée.

3. L'orthèse compressive de la revendication 1, dans laquelle la région à élasticité accrue est formée par tricotage d'un renfort élastique.

## Patentansprüche

1. Eine Kompressionsorthese vom Typ einer Stützstrumpfhose, welche einen Bauchteil (1) aus einem Kompressionsmaschennetz und zwei angefügte Beinteile (2) ebenso aus einem Kompressionsmaschennetz aufweist, wobei der Bauchteil vom einen Bereich (4) mit im wesentlichen dreieckiger Form aufweist, der sich zwischen der Gürtellinie und den Leistenbeugen erstreckt,
dadurch gekennzeichnet, daß der Bereich (4), der sich zwischen der Gürtellinie und den Leistenbeugen erstreckt, ein wenig verformbarer Bereich ist, der ein Plastron bildet, das eine Elastizität hoher Spannung in die zwei Richtungen aufweist, derart, daß dieser Bereich auf den Unterleib während der Post-Parfum Periode einen im wesentlichen konstanten Druck ausübt, der sich der Verformung des Unterleibs widersetzt, in Kombination mit einer Vaskulär-Kompression der unteren Glieder durch die Beinteile.

2. Kompressionsorthese nach Anspruch 1, bei welcher der Bercich erhöhter Elastizität durch Zusatz eines Gewebestücks mit zweidimensionaler verstärkter Elastizität gebildet ist.

3. Kompressionsorthese nach Anspruch 1, bei welcher der Bereich mit erhöhter Elastizität durch einen Stoff einer elastischen Verstärkung gebildet ist.

## Claims

1. A compressive orthosis of the elastic tights type, comprising an abdominal portion (1) of compressive knit and two adjoining leg portions (2), likewise of compressive knit, said abdominal portion including, in the front, a region (4) of substantially triangular shape extending between the waist and the groin, characterized in that said region (4) extending between the waist and the groin is a region with little deformability forming a starched front, providing high-pressure elasticity in both directions,
Whereby said region exerts on the abdomen during the post-partum period a substantially constant pressure in opposition to the deformation of the abdomen, in combination with vascular pressure on the lower limbs by means of the leg portions.

2. A compressive orthosis according to claim 1, in which the region of increased elasticity is formed by adding a patch of cloth having reinforced two-dimensional elasticity.

3. The compressive orthosis of claim 1, in which the region of increased elasticity is formed by knitting elastic reinforcement.
